(19) Europäisches Patentamt
European Patent Office
Office européen des brevets

(11) **EP 3 822 357 A1**

(12) **DEMANDE DE BREVET EUROPEEN**

(43) Date de publication:
**19.05.2021 Bulletin 2021/20**

(51) Int Cl.:
***C12P 19/14*** *(2006.01)*     ***C12N 9/42*** *(2006.01)*
***C12N 9/24*** *(2006.01)*     ***C12P 7/10*** *(2006.01)*
***C12R 1/885*** *(2006.01)*

(21) Numéro de dépôt: 20203529.1

(22) Date de dépôt: **23.10.2020**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(30) Priorité: **18.11.2019 FR 1912856**

(71) Demandeur: **IFP Energies nouvelles
92852 Rueil-Malmaison Cedex (FR)**

(72) Inventeurs:
• **BEN CHAABANE, Fadhel
92852 RUEIL-MALMAISON CEDEX (FR)**
• **JOURDIER, Etienne
92852 RUEIL-MALMAISON CEDEX (FR)**
• **AYMARD, Caroline
92852 RUEIL-MALMAISON CEDEX (FR)**
• **AUGIER, FREDERIC
92852 RUEIL-MALMAISON CEDEX (FR)**

(74) Mandataire: **IFP Energies nouvelles
Département Propriété Industrielle
Rond Point de l'échangeur de Solaize
BP3
69360 Solaize (FR)**

(54) **PROCÉDÉ DE PRODUCTION D'ENZYMES PAR UNE SOUCHE APPARTENANT À UN CHAMPIGNON FILAMENTEUX**

(57) La présente invention concerne un procédé de production d'enzymes cellulolytiques et/ou hémicellulolytiques par une souche de microorganisme appartenant à la famille des champignons filamenteux, ledit procédé comprenant les étapes suivantes :
(a) une première étape de croissance des champignons dans un milieu de culture aqueux, en présence d'au moins un substrat carboné de croissance, dans un bio-réacteur agité et aéré,
(b) une deuxième étape de production d'enzymes, à partir du milieu de culture aqueux obtenu à la première étape (a), en présence d'au moins un substrat carboné inducteur, induisant également la production d'hydrophobines, et on réintroduit dans une étape (d) au moins une partie des hydrophobines produites à l'étape (b) dans l'étape (a) de croissance.

Fig 1

EP 3 822 357 A1

## Description

### Domaine technique

L'invention est relative à un procédé de production de cellulases par un champignon filamenteux, nécessaire pour l'hydrolyse enzymatique de biomasse lignocellulosique mise en œuvre, par exemple, dans les procédés de production des jus sucrés dits de seconde génération (2G). Ces jus sucrés peuvent être utilisés pour produire d'autres produits par voie chimique ou voie biochimique/fermentaire (par exemple, des alcools comme des biocarburants du type éthanol, ou encore du butanol ou d'autres molécules, par exemple des solvants tels que l'acétone et d'autres molécules biosourcées...). Les cellulases peuvent également être employés dans d'autres procédés, notamment dans l'industrie chimique, papetière ou textile.

La mise au point de procédés économiquement viables de production de biocarburants de 2ème génération (dite « 2G »), si l'on prend cet exemple particulier de mise en œuvre, fait l'objet de nombreuses études. Ces derniers sont produits, notamment, à partir de substrats ligneux comme différents bois (feuillus et résineux, miscanthus, ou « TCR », qui est l'acronyme pour Taillis à Courte Rotation), des coproduits issus de l'agriculture (pailles de blé, pailles de riz, rafles de maïs, etc...) ou d'autres industries agroalimentaires, papetières, etc... Ils posent moins de problèmes de concurrence d'usage des terres agricoles avec l'alimentaire, par rapport aux biocarburants dits de première génération qui sont produits à partir de canne à sucre, maïs, blé ou betterave.

La biomasse lignocellulosique se caractérise par une structure complexe constituée de trois principales fractions: la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe. Parmi les trois polymères de base qui intègrent la biomasse lignocellulosique, la cellulose et l'hémicellulose sont ceux qui permettent la production de jus sucrés 2G.

De façon classique, le procédé de la transformation de la biomasse en biocarburant de type éthanol comprend plusieurs étapes : Le prétraitement permet de rendre la cellulose accessible aux enzymes qui sont des cellulases. L'étape d'hydrolyse enzymatique permet la transformation de la cellulose en sucres, comme le glucose, qui sont ensuite transformés en éthanol lors de l'étape de fermentation par en général la levure *Saccharomyces cerevisiae.*

Enfin l'étape de distillation va permettre de séparer et récupérer l'éthanol du moût de fermentation.
A noter, comme évoqué plus haut, que l'on peut aussi choisir d'arrêter le procédé à l'obtention des sucres monomères de type glucose, xylose, ...afin de les valoriser en tant que tels, ou encore les traiter différemment pour obtenir d'autres alcools ou molécules biosourcées.

### Technique antérieure

Les différentes études technico-économiques démontrent que la réduction du coût des cellulases est un des points-clés des procédés de production biologique d'éthanol à partir des matières premières lignocellulosiques. A l'heure actuelle, les cellulases industrielles sont principalement produites par un champignon filamenteux, *Trichoderma reesei,* en raison de son fort pouvoir de sécrétion.

Depuis les années 1970, la transformation de matériaux lignocellulosique en éthanol, après hydrolyse des polysaccharides constitutifs en sucres fermentescibles, a fait l'objet de nombreux travaux. On peut citer par exemple les travaux de référence du National Renewable Energy Laboratory (Process Design and Economics for Biochemical Conversion of Lignocellulosic Biomass to Ethanol, Humbird et al., NREL/TP-5100-57764, May 2011).

Les matériaux ligno-cellulosiques sont des matériaux cellulosiques, c'est-à-dire constitués à plus de 90% poids de cellulose, et/ou lignocellulosique, c'est-à-dire constitués de cellulose, d'hémicelluloses, qui sont des polysaccharides essentiellement constitués de pentoses et d'hexoses ainsi que de lignine, qui est une macromolécule de structure complexe et de haut poids moléculaire, à base de composés phénoliques.

Le bois, les pailles, les rafles de maïs sont les matériaux ligno-cellulosiques les plus utilisés, mais d'autres ressources, cultures forestières dédiées, résidus de plantes alcooligènes, sucrières et céréalières, produits et résidus de l'industrie papetière et des produits de transformation des matériaux ligno-cellulosiques sont utilisables. Ils sont constitués pour la plupart d'environ 35 à 50 % de cellulose, de 20 à 30 % d'hémicellulose et de 15 à 25 % de lignine.

Le procédé de transformation biochimique des matériaux ligno-cellulosiques en éthanol comprend une étape de prétraitement physico-chimique, suivie d'une étape d'hydrolyse enzymatique utilisant un cocktail enzymatique, d'une étape de fermentation éthanolique des sucres libérés, la fermentation éthanolique et l'hydrolyse enzymatique pouvant être conduites simultanément, et d'une étape de purification de l'éthanol.

Le cocktail enzymatique utilisé pour l'hydrolyse enzymatique est un mélange d'enzymes cellulolytiques (également appelées cellulases) et/ou hémicellulolytiques. Les enzymes cellulolytiques présentent trois grands types d'activités : endoglucanases, exoglucanases et cellobiases, ces dernières étant également appelées β-glucosidases. Les enzymes hémicellulolytiques présentent notamment des activités xylanases.

L'hydrolyse enzymatique est efficace et s'effectue dans des conditions douces. En revanche, le coût des enzymes reste très élevé, représentant de 20 à 50% du coût de transformation du matériau lignocellulosique en éthanol. De ce fait, beaucoup de travaux ont été conduits pour réduire ce coût : l'optimisation de la production d'enzymes d'abord, en sélectionnant les microorganismes hyper producteurs et en améliorant les procédés de production desdites enzymes, la diminution de la quantité d'enzymes en hydrolyse ensuite, en optimisant l'étape de prétraitement, en améliorant l'activité spécifique de ces enzymes, et en optimisant la mise en œuvre de l'étape d'hydrolyse enzymatique.

De nombreux travaux se sont attachés à comprendre les mécanismes d'action et d'expression du cocktail enzymatique. Le but est de faire sécréter le cocktail le plus approprié à l'hydrolyse des matériaux lignocellulosiques en modifiant les microorganismes.

*Trichoderma reesei* est le microorganisme le plus utilisé pour la production de cellulases. Les souches sauvages ont la faculté d'excréter, en présence d'un substrat inducteur, la cellulose par exemple, le complexe enzymatique considéré comme le mieux adapté à l'hydrolyse de la cellulose. Les enzymes du complexe enzymatique contiennent trois grands types d'activités : les endoglucanases, les exoglucanases et les cellobiases et d'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques. La nature du substrat carboné a une forte influence sur la composition du complexe enzymatique. C'est le cas des xyloses, qui, associé à un substrat carboné inducteur comme la cellulose ou le lactose, permet d'améliorer significativement l'activité dite xylanase. La régulation des gènes de cellulases sur différentes sources de carbone a été étudiée en détails. Ils sont induits en présence de cellulose, de ses produits d'hydrolyse , comme le cellobiose, ou de certains oligosaccharides comme le lactose ou le sophorose (cf. Ilmén et al., 1997; Appl.Environ. Microbiol. 63: 1298-1306.)

Les techniques de génétique classique par mutation ont permis la sélection de souches de *Trichoderma reesei* hyperproductrices de cellulases telles que les souches MCG77 (Gallo - brevet US 4,275,167), MCG 80 (Allen, A.L. et Andreotti, R.E., Biotechnol- Bioengi 1982, 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50).

Le procédé de production de cellulases par *Trichoderma reesei* a fait l'objet d'améliorations importantes en vue d'une extrapolation à l'échelle industrielle. Pour obtenir de bonnes productivités en enzymes, il est nécessaire d'apporter une source de carbone rapidement assimilable pour la croissance de *Trichoderma reesei,* et un substrat inducteur qui permet l'expression des cellulases et la sécrétion dans le milieu de culture. La cellulose peut jouer ces deux rôles ; cependant, elle est difficile d'utilisation au stade industriel et il a été proposé de la remplacer par des sources de carbone solubles, de type glucose, xylose ou lactose, le lactose jouant également le rôle de substrat inducteur. D'autres sucres solubles comme le cellobiose et le sophorose ont été décrits comme inducteurs, mais ils sont relativement onéreux pour être utilisés au stade industriel. On a aussi constaté que les productions de cellulases par *Trichoderma reesei,* avec des substrats solubles, sont très inférieures à celles obtenues sur cellulose en "batch". Ceci est dû à l'effet répresseur des sucres facilement assimilables, à forte concentration. L'alimentation en continu en mode fed-batch des substrats carbonés solubles a permis de lever la répression catabolique en limitant la concentration résiduelle dans les cultures et en optimisant la quantité de sucre permettant d'obtenir un meilleur rendement et une meilleure productivité enzymatique.

Le brevet FR-B-2 555 603 propose un protocole qui permet d'aboutir à une concentration en protéines de l'ordre de 35 à 40 g/L avec une productivité de l'ordre de 0,2 g/L/h et qui consiste en deux étapes : une première étape de croissance en mode "batch" où il est nécessaire d'apporter une source de carbone rapidement assimilable pour la croissance de Trichoderma reesei, puis une étape de production en mode "fed-batch" utilisant un substrat inducteur (exemple: le lactose) qui permette l'expression des cellulases et la sécrétion dans le milieu de culture. Le flux optimal appliqué est compris entre 35 et 45 mg.g-1.h-1 (milligrammes de substrat inducteur par milligramme de biomasse et par heure). On peut également citer le brevet EP-B-2 744 899 qui en propose une amélioration, en sélectionnant notamment un bioréacteur ayant un coefficient de transfert volumétrique d'oxygène kLa particulier, associé à une sélection particulière à la fois de la concentration en substrat carboné de croissance dans la première étape et d'un niveau de flux limitant de source de carbone dans la seconde étape.

Par ailleurs, le champignon filamenteux de type Trichoderma reesei est connu pour son caractère aérobie strict : il supporte mal le manque d'oxygène dissous. Il est possible de définir une concentration en oxygène dissous minimale permettant une culture satisfaisante, cette concentration étant généralement comprise entre 1 et 5 mg/L.

Comme détaillé dans l'article de 2012 de Gabelle J.-C., Jourdier E., Licht R.B., Ben Chaabane F., Henaut I., Morchain J., and Augier F. "Impact of rheology on the mass transfer coefficient during the growth phase of Trichoderma reesei in stirred bioreactors" (Chemical Engineering Science 75 (2012) 408-417), pour réaliser une culture dans les bonnes conditions d'oxygénation, le réacteur utilisé (appelé aussi fermenteur) est généralement conçu et opéré de façon à permettre de réaliser un transfert d'oxygène suffisant pour atteindre la concentration d'oxygène dissous minimale évoquée plus haut durant toute la culture. Or, tout au long du procédé de production d'enzymes, comprenant l'étape de croissance des champignons puis l'étape de production d'enzymes à proprement parlé, la partie la plus délicate pour respecter ce critère de concentration d'oxygène dissous minimal s'est avérée être la fin de l'étape de croissance, car c'est à la fois le moment où la demande en oxygène est la plus forte, et le moment où la viscosité du moût fermentaire tend à être la plus élevée.

Or il est connu que la viscosité impacte négativement le transfert d'oxygène (Gabelle et al., 2011 - Gabelle JC, Jourdier E, Licht RB, Ben Chaabane F, Henaut I, Morchain J, and Augier F (2012) Impact of rheology on the mass transfer coefficient during the growth phase of Trichoderma reesei in stirred bioreactors. Chemical Engineering Science 75, 408-417) : plus la concentration en biomasse augmente, plus le flux d'oxygène à transférer tend à augmenter, mais plus ce transfert est difficile à réaliser du fait de cette augmentation de viscosité, ce qui peut nécessiter d'adapter des conditions opératoires. Le flux d'oxygène transféré d'une phase gazeuse vers un liquide est conditionné par de nombreux paramètres, comme la pression partielle d'oxygène en phase gaz, la pression dans le réacteur, le débit d'air injecté, la concentration en oxygène dissous, et la puissance dissipée par une éventuelle agitation mécanique dans le fermenteur.

Généralement, le gaz injecté est de l'air, pour des raisons économiques. De plus, la pression ne peut être augmentée que de quelques bars, car au-delà la concentration en $CO_2$ dissous peut inhiber la culture. Ces deux leviers d'action ont un impact direct sur la rentabilité de la production des enzymes, car ils augmentent potentiellement la consommation énergétique, voire les investissements, des installations mettant en œuvre ce procédé.

Dans un fermenteur donné, ayant des capacités d'aération et d'agitation maximales bien connues, il n'est pas envisageable de dépasser une certaine concentration en biomasse en fin de croissance. En effet, l'aération et l'agitation une fois poussées à leur niveau maximal, une fois atteinte la concentration minimale d'oxygène dissous requise, toute augmentation de la concentration en biomasse induirait à la fois une augmentation de la demande en oxygène et une augmentation de la viscosité, conduisant à une diminution de la concentration en oxygène dissous, pouvant nuire au rendement global de la fermentation. On voit donc que la nécessité de ce transfert d'oxygène vers la phase liquide dans le fermenteur crée des contraintes industrielles / économiques impactant le rendement global de la production d'enzymes.

Il est par ailleurs connu de la demande de brevet EP-1 204 738 de modifier génétiquement les souches de champignons, notamment de type Trichoderma, et tout particulièrement leurs séquences ADN codant les hydrophobines, afin d'éviter que les souches ne sécrètent ces hydrophobines, et notamment les HFBII, jugées responsables de la formation de mousse. Cependant, toute solution impliquant des modifications génétiques est lourde à mettre en œuvre, puisqu'elle nécessite de procéder à ces modifications génétiques sur chacune des souches d'intérêt.

L'invention a alors pour but un procédé amélioré de production d'enzymes, visant notamment à remédier aux inconvénients précités. Elle a notamment pour but d'améliorer la productivité du procédé, notamment en améliorant/augmentant le transfert d'oxygène dans la phase liquide du réacteur le mettant en œuvre sans ajouter/en limitant toute contrainte supplémentaire sur les conditions opératoires ou la conception du réacteur.

L'invention a tout d'abord pour objet un procédé de production d'enzymes cellulolytiques et/ou hémicellulolytiques par une souche de microorganisme appartenant à la famille des champignons filamenteux, tel que ledit procédé comprend (par exemple consiste en) les étapes suivantes :

(a) une première étape de croissance des champignons dans un milieu de culture aqueux, en présence d'au moins un substrat carboné de croissance, dans un bioréacteur agité et aéré, notamment en phase batch,
(b) une deuxième étape de production d'enzymes, à partir du milieu de culture aqueux obtenu à la première étape (a), en présence d'au moins un substrat carboné inducteur, induisant également la production d'hydrophobines,

et où l'on réintroduit dans une étape (d) au moins une partie des hydrophobines produites à l'étape (b) dans l'étape (a) de croissance.

Les inventeurs ont en effet étudié le transfert d'oxygène entre une phase liquide (le milieu de culture), et une phase gazeuse (l'oxygène, ou plus généralement de l'air) venant apporter l'oxygène nécessaire aux champignons pour croître et produire les enzymes recherchées, ce transfert impliquant de nombreux paramètres physiques. Il s'est avéré qu'une grandeur de grande importance sur le transfert d'oxygène est la taille des bulles. En effet, plus les bulles sont petites, plus l'air injecté va générer de surface d'échange. C'est d'ailleurs principalement pour cette raison qu'un dispositif d'agitation est souvent mis en œuvre dans les fermenteurs, pour diminuer la taille des bulles et ainsi augmenter le transfert d'oxygène. Deux phénomènes gouvernent la taille des bulles dans un réacteur ou fermenteur gaz-liquide : la brisure des bulles et leur coalescence. Ils permettent respectivement de diminuer et d'augmenter la taille des bulles, et donc d'augmenter et de diminuer le transfert de matière. Ainsi, les inventeurs ont considéré que, si par un moyen particulier, le phénomène de coalescence venait à être diminué, cela permettrait d'augmenter le transfert de matière et donc la concentration en biomasse maximale atteignable dans un fermenteur donné, et cela tout en maintenant l'oxygène dissous au-dessus de la valeur minimale requise.

Il existe de nombreuses molécules permettant de bloquer partiellement la coalescence des bulles en milieu aqueux, tels que des tensio-actifs comme le Sodium Dodecyl Sulfate (SDS) ou le Bovine Serum Albumin (BSA). Ces molécules ont néanmoins l'inconvénient de générer un fort moussage dans ces milieux fermentaires, ce qui présente un vrai problème de conduite industrielle du procédé, et elles ont en plus un impact négatif en termes de cout de matières premières.

Enfin, les inventeurs ont observé que le champignon de type Trichoderma reesei produit de nombreuses protéines durant l'étape de croissance, et surtout pendant l'étape de production. Parmi celles-ci, la famille de molécules appelées hydrophobines s'est avérée d'un intérêt particulier. En effet ces molécules ont un impact important sur la coalescence, et donc la taille des bulles, et leur présence augmente sensiblement les performances de transfert. Néanmoins elles ont l'inconvénient d'être secrétées majoritairement durant l'étape de production, alors que le transfert gaz-liquide est un phénomène sensible surtout en étape de croissance, et non en production.

La présente invention a donc mis à profit cette propriété des hydrophobines, en les extrayant en fin de phase de production pour les réinjecter en phase de croissance. On vient en quelque sorte recycler les hydrophobines produites en phase de production dans la phase de croissance. Cette solution présente beaucoup d'avantages : les hydrophobines vont jouer leur rôle de limitant de coalescence en phase de croissance, ce qui va in fine permettre de réduire les couts liés aux moyens d'agitation (cout d'équipement et cout énergétique) et/ou d'avoir une concentration en champignons plus élevée pour un volume/un type de réacteur donné.

On souligne également que l'invention n'engendre pas de complication significative dans la conduite du procédé de production d'enzymes, et ne contraint pas à réaliser des modifications génétiques spécifiques sur les microorganismes.

L'invention propose différentes variantes/différents modes de réalisation pour extraire les hydrophobines du milieu de production, avec différents types de séparation possibles.

De préférence, le procédé selon l'invention prévoit après l'étape (b) de production d'enzymes et avant l'étape (d) de réintroduction des hydrophobines au moins une étape (c) de séparation des hydrophobines, notamment en phase liquide, qui est réalisée sur le milieu de culture de l'étape (b) de production, notamment au moyen d'une ou plusieurs filtrations successives dudit milieu de culture.

Selon une première variante, l'étape (c) de séparation des hydrophobines est réalisée par séparation directe d'au moins une partie des hydrophobines en phase liquide du milieu de culture de l'étape (b) de production. On sépare donc (par filtration par exemple) les hydrophobines directement du milieu de culture fermentaire, et on laisse ensemble les champignons (appelés alternativement biomasse cellulaire dans le présent texte) et les enzymes pour leur utilisation ultérieure conjointe dans un procédé de conversion de biomasse lignocellulosique : dans certaines configurations, en effet, l'hydrolyse enzymatique de la biomasse lignocellulosique peut être réalisée par mise en contact avec un mout fermentaire à partir du mélange des champignons et des enzymes qu'ils ont produites, sans avoir besoin de séparer les champignons des enzymes.

Selon une deuxième variante, le procédé prévoit, après l'étape (b) de production et avant l'étape (d) de réintroduction des hydrophobines, une séparation (c) comprenant une première sous-étape (c1) de séparation entre d'une part les champignons, et d'autre part le reste du milieu de culture, puis une deuxième sous-étape (c2) de séparation dudit reste du milieu de culture entre d'une part les hydrophobines, notamment en phase liquide, et d'autre part les enzymes. On prévoit donc dans cette variante de séparer d'abord les champignons du milieu de culture, par exemple par filtration, ce qui permet de récupérer un filtrat riche en enzymes et en hydrophobines, puis de séparer les enzymes des hydrophobines, ce qui permet de récupérer, si cette séparation se fait par filtration, un filtrat riche en hydrophobines (et pauvre en enzymes) qu'on va pouvoir utiliser, en tout ou partie, en étape de croissance, tandis que les enzymes ainsi isolées pourront être utilisées pour l'hydrolyse enzymatique de biomasse lignocellulosique.

Selon un mode de réalisation préféré, l'étape (c) de séparation des hydrophobines (soit du reste du milieu de culture, soit des enzymes selon les deux variantes mentionnés plus haut notamment) comprend au moins une opération d'ultrafiltration des hydrophobines du milieu liquide dans lesquelles elles se trouvent, afin d'isoler les hydrophobines dans le filtrat en phase liquide. Cette ultrafiltration est de préférence réalisée avec une membrane de filtration présentant un seuil de coupure compris entre 3 et 30 kDa, notamment compris entre 5 et 15 kDa. En effet, une caractéristique très avantageuse dans le cadre de la présente invention concernant les hydrophobines, et notamment la molécule HFBII, est que celles-ci sont bien plus petites que les enzymes de type cellulases, hémicellulases, environ 7kDa pour 30 à 100 kDa pour les cellulases notamment, ce qui rend la séparation de ces molécules par filtration/ultrafiltration tout-à-fait appropriée, même si toute autre technique connue de séparation peut aussi être utilisée : les membranes filtrent ainsi dans le rétentat tous les composants présentant une taille supérieure à celle des hydrophobines, que l'on peut alors isoler et recueillir dans le filtrat en phase liquide

De préférence, la sous-étape (c1) de séparation entre les champignons et le reste du milieu réactionnel selon la deuxième variante est réalisée par filtration, notamment par filtre presse, même si toute autre technique connue de séparation peut aussi être utilisée.

De préférence, le procédé selon l'invention comprend, de manière préférée consiste en, les étape a) de croissance, b) de production, c) de séparation, soit en une seule étape soit en particulier consistant en la sous-étape c1 puis la sous-étape c2, et d) de réintroduction des hydrophobines à l'étape a).

Quelle que soit la façon dont les hydrophobines sont séparées du milieu de culture, de préférence, les hydrophobines isolées après séparation (c) et avant réintroduction (d) dans le milieu de culture de l'étape (a) sont en phase liquide, avec stockage intermédiaire éventuel, dilution et/ou concentration éventuelle(s) intermédiaire(s). Leur manipulation et leur introduction dans l'étape (a) de croissance s'en trouvent facilitées, d'autant plus quand la phase liquide en question est une phase aqueuse issue d'une filtration du milieu de culture de l'étape (b) de production : cette phase aqueuse vient ainsi en appoint voire en remplacement de la phase aqueuse du milieu de culture de l'étape (a), ce qui revient à recycler l'eau du milieu de production vers le milieu de croissance.

Avantageusement, les hydrophobines produites à l'étape (b) de production que l'on réintroduit dans l'étape (a) de croissance sont majoritairement, notamment essentiellement, des hydrophobines de type II. Parmi les hydrophobines, la molécule HFBII est en effet, une molécule majoritaire et ayant un fort impact sur la taille des bulles.

L'étape (a) de croissance et/ou l'étape (b) de production peuvent s'opérer en mode batch, fed-batch ou continu, ou selon plusieurs de ces modes successivement.

On peut réintroduire dans l'étape (d) les hydrophobines dans le milieu de culture de l'étape (a) de croissance de différentes façons : en continu, en une fois (au début ou au cours de l'étape (a) de croissance) ou, ou de façon séquentielle pendant toute ou partie de la durée de ladite étape (a) de croissance. Comme évoqué plus haut, on peut prévoir un stockage intermédiaire, en prévoyant un réservoir tampon par exemple.

De préférence, on réintroduit des hydrophobines produites à l'étape (b) de production dans l'étape (a) de croissance sous forme d'un filtrat en phase aqueuse issu du milieu de culture de l'étape (b), l'eau du milieu de culture de l'étape (a) de croissance provenant en tout ou partie dudit filtrat.

De préférence, on réintroduit des hydrophobines dans l'étape (a) de croissance en solution dans un milieu aqueux à une concentration comprise entre 10 et 400 mg/l, de préférence entre 50 et 200 mg/l. Ainsi, soit, quand elles sont extraites du milieu de culture par filtration, elles sont déjà dans cette gamme de concentration dans le filtrat aqueux obtenu et à une concentration appropriée, soit ce filtrat peut, avant réintroduction dans le milieu de croissance, être dilué ou concentré, pour atteindre cette gamme ou atteindre une valeur différente de concentration dans cette gamme.

De préférence, lors de la première étape (a) de croissance, la concentration en substrat carboné de croissance est choisie comprise entre 15 et 100 g/l, notamment entre 15 ou 20 et 60 g/l.

De préférence, la deuxième étape (b) de production est opérée avec un flux limitant de substrat carboné inducteur, notamment compris entre 30 et 140 mg.g-1.h-1, (c'est-à-dire entre 30 et 140 milligrammes par gramme de biomasse et par heure), de préférence entre 35 et 45 mg.g-1.h-1, et de préférence avec une solution aqueuse de substrat(s) carboné(s) à une concentration comprise entre 200 et 700 g/l. Cette solution de substrat(s) carboné(s) comprend au moins un substrat carboné inducteur, qui peut être choisi parmi le lactose, le sophorose, la cellulose, du cellobiose, un marc cellulosique ou un mélange d'au moins deux de ceux-ci.

Avantageusement, la souche utilisée est une souche de Trichoderma reesei ou de Trichoderma reesei modifiée par mutation sélection ou recombinaison génétique. Il peut s'agir notamment des souches CL847, RutC30, MCG77, ou MCG80 mentionnées plus haut. Mais, bien sûr et contrairement à l'enseignement de la demande de brevet EP-1 204 738 précitée, les mutations envisagées dans le cadre de l'invention, n'ont pas pour but, bien au contraire, d'empêcher la formation d'hydrophobines lors de sa croissance.

Optionnellement, le procédé selon l'invention peut comprendre une étape intermédiaire entre l'étape (a) et l'étape (b), cette étape intermédiaire étant une étape de dilution du milieu de culture obtenu à l'étape (a) de croissance.

En outre, l'étape (a) de croissance et l'étape (b) de production peuvent être réalisées dans le même bioréacteur ou dans deux réacteurs distincts, avec transfert du milieu réactionnel d'un réacteur à l'autre. Le premier cas est le plus simple, avec un seul réacteur on évite d'avoir à transférer le milieu réactionnel. Le second cas permet d'adapter précisément les caractéristiques et équipements de chacun des bioréacteurs en fonction des besoins de chacune des étapes. On peut avoir recours à différents types de bioréacteurs, dont des colonnes à bulles par exemple.

L'invention a aussi pour objet l'utilisation des enzymes obtenues par le procédé décrit plus haut pour l'hydrolyse enzymatique de biomasse cellulosique/hémicellulosique terrestre ou marine.

L'invention a également pour objet l'installation de production mettant en œuvre le procédé décrit plus haut, qui peut comprendre un seul bioréacteur pour les étapes de croissance et de production, ou deux bioréacteurs distincts et qui est équipée de moyens de séparation, notamment de filtration aptes à isoler les hydrophobines produites en phase de production, notamment de type moyen d'ultra-filtration.

On peut réintroduire les hydrophobines directement depuis un réacteur vers un autre, notamment quand on en utilise deux réacteurs distincts pour chacune des étapes de croissance et de production.

On peut prévoir un contenant intermédiaire dans lequel on vient stocker les hydrophobines prélevées en phase de production, pour les réintroduire ultérieurement en phase de croissance (dans le même réacteur ou un réacteur distinct).

On peut aussi prévoir une solution intermédiaire, avec pour partie un stockage temporaire des hydrophobines prélevées en phase de production pour une utilisation ultérieure, et pour partie une réintroduction directe dans un réacteur en phase de croissance.

L'invention sera ci-après décrite plus en détails à l'aide d'exemples de réalisation non limitatifs.

Liste des figures

Fig 1

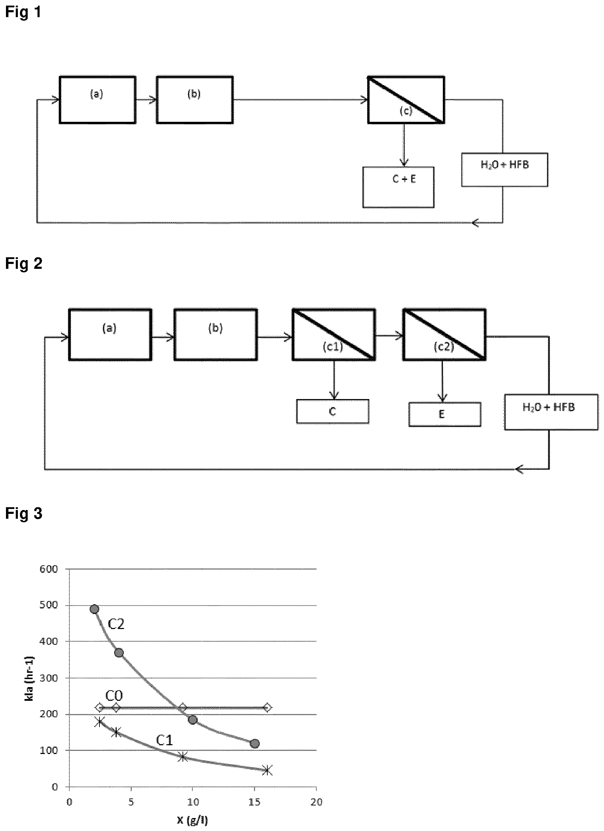

La figure 1 représente le schéma-bloc d'un procédé de production d'enzymes selon une première variante de l'invention.

Fig 2
La figure 2 représente le schéma-bloc d'un procédé de production d'enzymes selon une deuxième variante de l'invention.

Fig 3
La figure 3 est un graphe représentant en abscisse la concentration en champignons (biomasse cellulaire) en g/l, en en ordonnée le kLa correspondant en $h^{-1}$ pour de l'eau (courbe C0), et pour l'exemple 1 et l'exemple 2 décrits ci-après (respectivement courbes C1 et C2).

**Description des modes de réalisation**

La figure 1 correspond à la première variante du procédé de production d'enzymes selon l'invention. Les molécules de limitation de coalescence sont des hydrophobines produites lors de la phase de limitation en substrat carboné. Le procédé comporte :

- une étape (a) de croissance des champignons
- puis une étape (b) de croissance
- selon l'invention on ajoute une étape (c) de séparation par ultrafiltration, permettant d'obtenir d'une part un rétentat C+ E riche en champignons et en enzymes qu'ils ont produites, et d'autre part un filtrat comprenant de l'eau (le milieu de culture des étapes (a) et (b) est aqueux) enrichi en hydrophobines. Ce filtrat est ensuite réinjecté dans l'étape (a) de croissance. Il peut, avant réinjection, être dilué ou plus fréquemment concentré, et il peut aussi être stocké temporairement.

Dans cette variante, on filtre donc les hydrophobines directement du milieu fermentaire, et on laisse ensemble les champignons et les enzymes. Cette variante est particulièrement intéressante quand le procédé aval utilisant les enzymes peut exploiter ces enzymes sans séparation préalable des champignons : une seule séparation est nécessaire à mettre en œuvre l'invention.

La figure 2 correspond à une deuxième variante du procédé selon l'invention. On retrouve l'étape (a) de croissance puis l'étape (b) de production. Ici, on prévoit deux séparations successives selon l'invention : d'abord une séparation (c1), par exemple par filtration avec un filtre-presse, permettant de séparer d'une part un rétentat C riche en champignons, d'autre part un filtrat enrichi en enzymes et en hydrophobines. Puis ce filtrat subit une séparation (c2) par ultrafiltration permettant de récupérer d'une part un rétentat enrichi en enzymes, et d'autre part un filtrat comprenant de l'eau et enrichi en hydrophobines, ce filtrat étant ensuite, comme dans la variante précédente, réinjecté dans l'étape (a) de croissance.

Ici, on a donc séparé les enzymes du reste du milieu de culture, ce qui est intéressant quand le procédé aval utilise ces enzymes sous forme pure ou pour les commercialiser.

Dans ces deux variantes notamment, mais pour toute autre variante de l'invention, on a une séparation à l'étape (b) de production. A noter que cette séparation se fait préférentiellement en fin d'étape (b), et que cette réinjection se fait préférentiellement en début d'étape (a). Mais qu'il est également possible d'opérer la séparation par d'autres méthodes que par filtration, et il est également possible que la séparation soit réalisée tout au long de l'étape (b) ou sur une partie seulement de sa durée. De même, la réinjection des hydrophobines à l'étape (a) peut aussi se faire progressivement sur tout ou partie de la durée de l'étape (a).

On réintroduit de préférence les hydrophobines sous forme liquide, aqueuse (on les obtient directement sous cette forme en faisant les séparations par filtration). Après ajustement de la concentration en phase liquide, cette phase liquide peut même entièrement remplacer l'eau utilisée pour le milieu de culture de l'étape (a) de croissance.

Exemples de réalisation

Deux croissances comparatives de *Trichoderma reesei* en bioréacteur de 30 L ont été réalisées : La première est réalisée avec un milieu de culture conventionnel, et la deuxième est réalisée en remplaçant l'eau par un filtrat riche en hydrophobines issu d'une production précédente. Ce filtrat a été obtenu en filtrant le milieu de culture par ultrafiltration avec des membranes ayant un seuil de coupure de 10 kDa, il a ensuite été concentré.

Des mesures de rhéologie et de kLa sont réalisées dans les deux cas. Dans le premier cas, on voit que la viscosité affecte fortement le transfert d'oxygène (comme démontré dans l'article de 2012 de Gabelle J.-C. précité). Dans le deuxième cas, malgré la viscosité élevée, l'apport d'hydrophobines a un impact positif sur le kLa du milieu qui devient équivalent, voire supérieur, à celui de l'eau, obtenu dans les mêmes conditions d'agitation et d'aération.

La mesure de rhéologie est utilisée selon la méthode décrite dans l'article suivant : Nicolas Hardy, Frédéric Augier, Alvin W. Nienow, Catherine Béai, Fadhel Ben Chaabane. Scale-up agitation criteria for Trichoderma reesei fermentation: Chemical Engineering Science, Elsevier, 2017, 172, pp.158-168(10.1016/j.ces.2017.06.034).

Le calcul de kLa est réalisé par la méthode connue des bilans gazeux et décrite dans l'article précité : Gabelle JC, Jourdier E, Licht RB, Ben Chaabane F, Henaut I, Morchain J, and Augier F (2012) Impact of rheology on the mass transfer coefficient during the growth phase of Trichoderma reesei in stirred bioreactors. Chemical Engineering Science 75, 408-417. Le réacteur utilisé est un bioréacteur de 30L. Sa configuration est décrite dans le même article.

Exemple 1 (exemple comparatif - préliminaire à l'invention)

La croissance de Trichoderma reesei est effectuée dans le fermenteur de 30 L précité, agité mécaniquement avec un volume utile de 20 L. Le milieu minéral a la composition suivante : KOH 1,66 g./L, $H_3PO_4$ 85 % 2 mL/L, $(NH_4)_2SO_4$ 2,8 g/L, $MgSO_4$, 7 $H_2O$ 0,6 g/L, $CaCl_2$ 0,6 g/L, $MnSO_4$ 3,2 mg/L, $ZnSO_4$, 7 $H_2O$ 2,8 mg/L, $CoCl_2$ 10 4,0 mg/L, $FeSO_4$, 7 $H_2O$ 10 mg/L, Corn Steep 1,2 g/L, anti-mousse 0,5 mL/L. (qui sera au moins en partie consommé par le microorganisme) . De l'eau de ville est utilisée pour diluer les différents éléments du milieu et remplir le réacteur de façon à avoir un volume final de 20 L. Le fermenteur contenant le milieu minéral est stérilisé à 120 °C pendant 20 minutes, la source carbonée glucose est stérilisée à part à 120°C pendant 20 minutes puis ajoutée stérilement dans le bioréacteur de façon à avoir une concentration finale de 50 g/L. Le fermenteur est ensemencé avec une préculture liquide de 1L de la souche de *Trichoderma reesei* CL847. Le milieu minéral de la préculture est identique à celui du fermenteur, mis à part l'addition de phtalate de potassium à 5 g.L$^{-1}$ pour tamponner le pH du milieu. La croissance du champignon en préculture est faite en utilisant le glucose comme substrat carboné, à la concentration de 30 g.L$^{-1}$. La croissance de l'inoculum dure de 2 à 3 jours, et est effectuée à 28 °C dans un incubateur agité. Le transfert vers le fermenteur est réalisé quand la concentration résiduelle en glucose est inférieure à 15 g/L.

L'étape de croissance est effectuée pendant 50 heures dans le bioréacteur de 30 L agité à une température de 27 °C et un pH de 4,8 (régulé par de l'ammoniaque 5,5 M). L'aération est de 0,5 vvm (volume/volume/minute), et le pourcentage de la concentration d'oxygène dissous par rapport à la saturation dans le milieu liquide est régulé à 40 %. Le fermenteur est équipé d'un agitateur à 2 mobiles à pales droites inclinées, tournant à une vitesse de 1200 tr/min.

Des prélèvements réguliers sont réalisés pour suivre la rhéologie du milieu et la concentration en biomasse cellulaire. N'ayant pas d'éléments insolubles dans le milieu de culture, le poids sec, déterminé par filtration et séchage jusqu'à poids constant, représente la masse de champignons appelée aussi biomasse cellulaire. Un analyseur en sortie du bioréacteur permet de suivre la composition du gaz en $O_2$ et $CO_2$.

Les bilans gazeux permettent de calculer en continu la vitesse de consommation d'$O_2$, r$O_2$ et le kLa : A l'état pseudo-stationnaire, on a

$$rO_2 = Qin*\%O_2in - Qout*\%O_2out$$

$$rCO_2 = Qout*\%CO_2out - Qin*\%CO_2in$$

Avec:

    Qin : débit d'air en entrée en mol/h
    Qout : débit d'air en sortie en mol/h
    $\%O_2in$ : % molaire d'$O_2$ en entrée
    $\%O_2out$ : % molaire d'$O_2$ en sortie
    $\%CO_2in$ : % molaire de $CO_2$ en entrée
    $\%CO2out$ : % molaire de $CO_2$ en sortie

La r$O_2$ est utilisée pour calculer le kLa de culture grâce à la combinaison des deux bilans matière $O_2$ sur la phase liquide et la phase gazeuse à l'état pseudo-stationnaire :

$$kLa(t) = rO_2/(O_2*-O_{2L})$$

Avec :

    $O_2*$ : concentration d'$O_2$ à saturation
    $O_{2L}$ : Concentration d'oxygène dans le liquide

Selon la loi de Henry, la concentration maximale d'un gaz en solution, en équilibre avec une atmosphère contenant ce gaz, est proportionnelle à la pression partielle de ce gaz en ce point.
On a ainsi pour le cas de $O_2$ :

$$O_2* \ (mol/m^3) = 1,25 \ pO \ (bar)$$

Avec :
pO : pression partielle d'$O_2$
Il est à noter que la pression partielle d'$O_2$ est égale au produit de la fraction molaire de l'$O_2$ dans le gaz et de la pression. L'$O_2*$ dans un fermenteur industriel est donc maximal en bas de réacteur (pression maximale et $\%O_2$ en entrée de 21%) et minimal en haut (pression de ciel et $\%O_2$ dans le gaz en sortie). Il est calculé à chaque instant de l'expérience, car la composition en $O_2$ du gaz en sortie diminue du fait de la consommation d'$O_2$ par le microorganisme. Dans le cas d'un réacteur de laboratoire, la différence de pression entre le haut et le bas du réacteur est négligeable. La concentration en oxygène dans le liquide est calculée grâce à la mesure de la sonde $pO_2$ qui donne un pourcentage d'$O_2$ par rapport à la saturation.

Ainsi, durant la croissance du champignon, il a été possible de mesurer à différents instants la concentration en biomasse cellulaire (X), la viscosité ($\mu$a) du milieu fermentaire sous un cisaillement de 10 s$^{-1}$, et un coefficient de transfert gaz-liquide kLa en h$^{-1}$.

Les mesures de kLa sont comparées à une mesure réalisée dans de l'eau, à même débit d'air et même vitesse d'agitation. Le tableau 1 ci-dessous présente les résultats pour les coefficients de transfert obtenus durant la fermentation selon l'art antérieur.

**Tableau 1**

| X (g/L) | $\mu$a (10 s$^{-1}$) | kLa (h$^{-1}$) | kLa eau (h$^{-1}$) |
|---|---|---|---|
| 2.5 | 0,03 | 180 | 217 |
| 3.8 | 0,055 | 150 | 217 |
| 9.2 | 0,21 | 82 | 217 |
| 16 | 0,48 | 45 | 217 |

La phase de production a ensuite été réalisée à pH 4 à 25°C, avec une concentration en lactose de 220 g/L, correspondant à un débit spécifique de lactose de fed-batch de 45 mg par gramme de biomasse et par heure.

Exemple 2 (selon l'invention)

Une expérience est réalisée dans les mêmes conditions, mais en remplaçant l'eau par une solution issue d'une expérience précédente de production de cellulases sur laquelle une concentration de 100 mg/L d'hydrophobines HFBII (seules les HFBII ont été dosées, il est possible que les hydrophobines utilisées comprennent aussi d'autres types d'hydrophobines, de façon minoritaire) a été déterminée par HPLC (acronyme pour« High Performance Liquide Chromatography », c'est-à-dire chromatographie liquide à haute performance) avec une Colonne Wide Pore C5 (150 x 2,1 mm ; 5 $\mu$m) et une détection UV. Une filtration a été réalisée à la fin de cette expérience pour séparer le champignon des cellulases, et une ultrafiltration avec des membranes ayant une porosité de 10 kDa (Les membranes préconisées sont de type UFX 10 pHt commercialisées par la société Alfa Laval. Il convient de respecter les données fournisseurs pour leur mise en œuvre (conditions de pression, température ...). Pour le produit, il est préférable de ne pas dépasser 30°C lors de cette ultrafiltration, 20 à 25 °C est une gamme de températures préférée. Après ultrafiltration, les perméats contiennent les hydrophobines. L'analyse de l'échantillon moyen de perméat, permet de doser l'échantillon. Les débits obtenus sont de 20 l/h/m$^2$ de membrane. L'ultrafiltration permet de concentrer les cellulases et de récupérer dans le filtrat un milieu contenant des hydrophobines. La concentration des hydrophobines a ensuite de nouveau été mesurée par HPLC et est proche de 100 mg/L. C'est cette solution qui a été utilisée comme eau de dilution dans le bioréacteur de 30 L. A noter qu'un complément d'eau a été ajouté, diluant ainsi de 25% la concentration en hydrophobines au démarrage (temps T0) de l'expérience. Les conditions opératoires sont les mêmes que pour l'exemple 1.

La phase de production est également conduite, après la phase de croissance, dans les mêmes conditions opératoires que pour l'exemple 1.

La viscosité et le coefficient de transfert sont mesurés durant la croissance du champignon. Les résultats obtenus sont présentés dans le tableau 2 qui indique la concentration en biomasse cellulaire (X), la viscosité du milieu fermentaire sous un cisaillement de 10 s$^{-1}$ ($\mu$a), et un coefficient de transfert gaz-liquide kLa en h$^{-1}$ comme dans le tableau 1 précédent.

**Tableau 2**

| X (g/L) | $\mu$a (10 s$^{-1}$) | kLa (h$^{-1}$) | kLa eau (h$^{-1}$) |
|---|---|---|---|
| 2 | 0,021 | 480 | 217 |
| 4 | 0,06 | 370 | 217 |
| 10 | 0,24 | 185 | 217 |
| 15 | 0,44 | 120 | 217 |

Une comparaison des performances obtenues selon les deux exemples 1 et 2 est représentée sur le graphe de la figure 3 : On remarque que les coefficients de transfert kLa obtenus selon l'invention (courbe C1 pour l'exemple 1 comparatif, courbe C2 pour l'exemple 2 selon l'invention) sont au moins deux fois plus élevés que selon l'art antérieur. Selon la concentration en biomasse, le coefficient de transfert est tantôt inférieur tantôt supérieur à celui mesuré dans de l'eau (courbe C0) aux même conditions d'aération et de agitation.

Ces exemples montrent que le transfert d'oxygène est grandement facilité par l'invention. Cet avantage peut être exploité de différentes manières, et notamment :

- pour abaisser la vitesse d'agitation dans le bioréacteur, pour atteindre le coefficient de transfert cible en consommant moins d'énergie,
- pour abaisser le débit d'air dans le bioréacteur pour les mêmes raisons,
- pour réaliser des cultures avec plus de biomasse cellulaire, tout en restant au-dessus d'une concentration cible minimale en oxygène dissous, ce qui permet d'augmenter la productivité des bioréacteurs (appelés aussi fermenteurs).

Il a en outre été vérifié que les performances de production d'enzymes des deux exemples, évaluées en fin de phase de production, sont les mêmes ou quasiment les mêmes pour les deux exemples : ces avantages ne s'obtiennent donc pas au détriment des performances, du rendement de production, du procédé.

Comme le coefficient de transfert kLa est proportionnel à la puissance dissipée par unité de volume (P/V, exprimée en W/m$^3$), dans une gamme de puissance par unité de volume P/V comprise entre 0,4 et 0,5 kW/m$^3$ (selon l'article précité de Gabelle et al. 2012), l'augmentation du kLa d'un facteur 2 au moins permet d'économiser entre 75 et 85% de la puissance dissipée par unité de volume, ce qui est un gain énorme à l'échelle industrielle.

A noter par ailleurs que si les exemples 1 et 2 utilisent des sources carbonées spécifiques pour la croissance et pour la production, l'invention s'applique naturellement avec d'autres sources carbonées, telles que des sucres solubles comme le lactose, le glucose ou le xylose. Le substrat carboné de croissance peut être choisi parmi le lactose, le glucose, le xylose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique. Le substrat carboné inducteur est choisi de préférence parmi le lactose, le cellobiose, le sophorose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique. Ce type de résidu /extrait peut donc être utilisé aussi comme source de carbone totale, c'est-à-dire à la fois pour la croissance du microorganisme et l'induction du système d'expression. Cette source de carbone est utilisable plus particulièrement par les souches améliorées génétiquement et notamment les souches recombinantes.

De même, l'invention s'applique également dans les conditions opératoires différentes de celles expressément prévues aux exemples. Ainsi, le pH et la température, pour l'étape de croissance et l'étape de production, peuvent être les suivantes :

- pH entre 3,5 et 4,4 ;
- température entre 20 et 35 °C.

La vvm (taux d'aération exprimé en volume d'air par volume de milieu réactionnel et par minute) appliquée durant le procédé est comprise entre 0,3 et 1,5 min$^{-1}$ et la rpm (vitesse de rotation) doit permettre de réguler le pourcentage de de la concentration d'oxygène dissous par rapport à la saturation dans le milieu liquide entre 20% et 60% de O$_2$. On choisit de préférence une aération de 0,3 à 0,5 min$^{-1}$, et une agitation permettant de réguler le pourcentage de concentration d'oxygène dissous entre 30 % et 40% de O$_2$.

Selon sa nature, le substrat carboné choisi pour l'obtention de la biomasse est introduit dans le fermenteur avant stérilisation, ou est stérilisé séparément et introduit dans le fermenteur après stérilisation. La concentration en substrat carboné est comprise entre 200 et 700 g/L selon le degré de solubilité des substrats carbonés utilisés (notamment en ce qui concerne le substrat inducteur qui fait partie de ces substrats carbonés).

## Revendications

1. Procédé de production d'enzymes cellulolytiques et/ou hémicellulolytiques par une souche de microorganisme appartenant à la famille des champignons filamenteux, ledit procédé comprenant les étapes suivantes :

   (a) une première étape de croissance des champignons dans un milieu de culture aqueux, en présence d'au moins un substrat carboné de croissance, dans un bioréacteur agité et aéré, notamment en phase batch,
   (b) une deuxième étape de production d'enzymes, à partir du milieu de culture aqueux obtenu à la première étape (a), en présence d'au moins un substrat carboné inducteur, induisant également la production d'hydrophobines,

   **caractérisé en ce qu'**on réintroduit dans une étape (d) au moins une partie des hydrophobines produites à l'étape (b) dans l'étape (a) de croissance.

2. Procédé selon la revendication précédente, **caractérisé en ce que** ledit procédé prévoit après l'étape (b) de production d'enzymes et avant l'étape (d) de réintroduction des hydrophobines au moins une étape de séparation (c) des hydrophobines, notamment en phase liquide, qui est réalisée sur le milieu de culture de l'étape (b) de production, notamment au moyen d'une ou plusieurs filtrations successives dudit milieu de culture.

3. Procédé selon la revendication précédente, **caractérisé en ce que** l'étape (c) de séparation des hydrophobines est réalisée par séparation directe d'au moins une partie des hydrophobines en phase liquide du milieu de culture de l'étape (b) de production.

4. Procédé selon la revendication 2, **caractérisé en ce qu'**il prévoit, après l'étape (b) de production et avant l'étape (d) de réintroduction des hydrophobines, une séparation (c) comprenant une première sous-étape (c1) de séparation entre d'une part les champignons, et d'autre part le reste du milieu de culture, puis une deuxième sous-étape (c2) de séparation dudit reste du milieu de culture entre d'une part les hydrophobines, notamment en phase liquide, et d'autre part les enzymes.

5. Procédé selon l'une des revendications 2 à 4, **caractérisé en ce que** l'étape de séparation (c) comprend au moins une opération d'ultrafiltration des hydrophobines du milieu liquide dans lesquelles elles se trouvent, afin d'isoler les hydrophobines dans le filtrat en phase liquide, ladite ultrafiltration étant notamment réalisée avec une membrane de filtration présentant un seuil de coupure compris entre 3 et 30 kDa, notamment compris entre 5 et 15 kDa.

6. Procédé selon la revendication 4, **caractérisé en ce que** la sous-étape (c1) de séparation entre les champignons et le reste du milieu réactionnel est réalisée par filtration, notamment par filtre presse.

7. Procédé selon l'une des revendications 2 à 6, **caractérisé en ce que** les hydrophobines isolées après séparation (c) et avant réintroduction (d) dans le milieu de culture de l'étape (a) sont en phase liquide, avec stockage, dilution et/ou concentration éventuelle(s) intermédiaire(s).

8. Procédé selon l'une des revendications précédentes, **caractérisé en ce que** les hydrophobines produites à l'étape (b) de production que l'on réintroduit dans l'étape (a) de croissance sont majoritairement, notamment essentiellement, des hydrophobines de type II.

9. Procédé selon l'une des revendications précédentes, **caractérisé en ce que** l'étape (a) de croissance et /ou l'étape (b) de production s'opèrent en mode batch, fed-batch ou continu ou selon plusieurs de ces modes successivement.

10. Procédé selon l'une des revendications précédentes, **caractérisé en ce qu'**on réintroduit dans l'étape (d) les hydrophobines dans le milieu de culture de l'étape (a) de croissance en continu, en une fois ou de façon séquentielle pendant la durée de ladite étape (a) de croissance.

11. Procédé selon l'une des revendications précédentes, **caractérisé en ce qu'**on réintroduit des hydrophobines produites à l'étape (b) de production dans l'étape (a) de croissance sous forme d'un filtrat en phase aqueuse issu du milieu de culture de l'étape (b), l'eau du milieu de culture de l'étape (a) de production provenant en tout ou partie dudit filtrat.

12. Procédé selon l'une des revendications précédentes, **caractérisé en ce qu'**on réintroduit des hydrophobines dans l'étape (a) de croissance en solution dans un milieu aqueux à une concentration comprise entre 10 et 400 mg/l, de préférence entre 50 et 200 mg/l.

13. Procédé selon l'une des revendications précédentes, **caractérisé en ce que**, lors de l'étape (a) de croissance, la concentration en substrat carboné de croissance est comprise entre 15 et 100 g/l, et en que l'étape (b) de production est opérée avec un flux limitant de substrat carboné inducteur, notamment compris entre 30 et 140 mg.g-1.h-1, et de préférence entre 35 et 45 mg.g-1.h-1, et de préférence avec une solution aqueuse de substrat(s) carboné(s) à une concentration comprise entre 200 et 700 g/l.

14. Procédé selon l'une des revendications précédentes, **caractérisé en ce que** la souche de microorganisme utilisée est une souche de Trichoderma reesei ou de Trichoderma reesei modifiée par mutation sélection ou recombinaison génétique.

15. Utilisation des enzymes obtenues par le procédé selon l'une des revendications précédentes pour l'hydrolyse enzymatique de biomasse cellulosique/hémicellulosique terrestre ou marine.

**Fig 1**

**Fig 2**

**Fig 3**

Europäisches
Patentamt

European
Patent Office

Office européen
des brevets

# RAPPORT DE RECHERCHE EUROPEENNE

Numéro de la demande

EP 20 20 3529

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 2 371 950 A1 (IFP ENERGIES NOUVELLES [FR]) 5 octobre 2011 (2011-10-05) * colonne 1, alinéa [0001] * * colonne 2, alinéa [0014] * * colonne 3, alinéa [0016]; revendications 1-10 * | 15 | INV. C12P19/14 C12N9/42 C12N9/24 C12P7/10 C12R1/885 |
| X | WO 2017/174378 A1 (IFP ENERGIES NOW [FR]; ARD SA [FR]; AGRONOMIQUE INST NAT RECH [FR]) 12 octobre 2017 (2017-10-12) * page 1, lignes 1-10 * * page 6, ligne 1 - page 11, ligne 28; revendication 12 * | 15 | |
| X,D | WO 2013/026964 A1 (IFP ENERGIES NOUVELLES [FR]; AGRONOMIQUE INST NAT RECH [FR] ET AL.) 28 février 2013 (2013-02-28) * page 1, lignes 2-6 * * page 5, lignes 1-11 * * page 7, lignes 18-21 * | 15 | |

-/--

| | DOMAINES TECHNIQUES RECHERCHES (IPC) |
|---|---|
| | C12P C12N C12R |

Le présent rapport a été établi pour toutes les revendications

| Lieu de la recherche | Date d'achèvement de la recherche | Examinateur |
|---|---|---|
| Berlin | 6 avril 2021 | Mateo Rosell, A |

CATEGORIE DES DOCUMENTS CITES

X : particulièrement pertinent à lui seul
Y : particulièrement pertinent en combinaison avec un
  autre document de la même catégorie
A : arrière-plan technologique
O : divulgation non-écrite
P : document intercalaire

T : théorie ou principe à la base de l'invention
E : document de brevet antérieur, mais publié à la
  date de dépôt ou après cette date
D : cité dans la demande
L : cité pour d'autres raisons

& : membre de la même famille, document correspondant

EPO FORM 1503 03.82 (P04C02)

page 1 de 3

Europäisches
Patentamt

European
Patent Office

Office européen
des brevets

**RAPPORT DE RECHERCHE EUROPEENNE**

Numéro de la demande

EP 20 20 3529

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | LINDER MARKUS ET AL: "The hydrophobins HFBI and HFBII from Trichoderma reesei showing efficient interactions with nonionic surfactants in aqueous two-phase systems", BIOMACROMOLECULES, AMERICAN CHEMICAL SOCIETY, US, vol. 2, no. 2, 1 juillet 2001 (2001-07-01), pages 511-517, XP002512510, ISSN: 1525-7797, DOI: 10.1021/BM0001493 [extrait le 2001-05-17] * page 512, colonne de gauche, alinéa 4 * * page 512, colonne de droite, alinéa 2 * * page 513, colonne de gauche, alinéa 2 - colonne de droite, alinéa 3; tableaux 1-3 * * page 515, colonne de gauche, dernier alinéa; figure 2 * ----- | 1-14 | |
| A | US 2012/252066 A1 (HENG MENG H [US] ET AL) 4 octobre 2012 (2012-10-04) * page 1, alinéa [0009] - page 2, alinéa [0013] * * page 2, alinéa [0018] * * page 9, alinéa [0087]-[0089] * * page 11, alinéa [105]; exemples 1-7 * * revendications 1,2,4,14 * ----- | 1-14 | |
| X | DE 10 2010 063942 A1 (UNIV DRESDEN TECH [DE]) 28 juin 2012 (2012-06-28) * page 3, alinéa [0001] * * page 3, alinéa [0011] - page 4, alinéa [0012] * * page 5, alinéa [0030] - page 8, alinéa [0058] * ----- | 1-14 | |

DOMAINES TECHNIQUES RECHERCHES (IPC)

-/--

Le présent rapport a été établi pour toutes les revendications

| Lieu de la recherche | Date d'achèvement de la recherche | Examinateur |
|---|---|---|
| Berlin | 6 avril 2021 | Mateo Rosell, A |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

................................................................

& : membre de la même famille, document correspondant

EPO FORM 1503 03.82 (P04C02)

page 2 de 3

Europäisches
Patentamt

European
Patent Office

Office européen
des brevets

**RAPPORT DE RECHERCHE EUROPEENNE**

Numéro de la demande

EP 20 20 3529

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2011/019686 A1 (DANISCO US INC [US]; BODO MICHAEL [US]; HENG MENG H [US]) 17 février 2011 (2011-02-17) * page 1, alinéa [002] * * page 5, alinéa [0032] - page 6, alinéa [0036] * * page 11, alinéa [0063] - page 14, alinéa [0073] * * exemples 1-6 * ----- | 1-14 | |
| A | WO 2012/142557 A1 (DANISCO US INC [US]; SCHELLE MICHAEL [US]) 18 octobre 2012 (2012-10-18) * page 1, alinéa [003] * * pages 2-3, alinéa [0011]; exemple 1 * ----- | 1-14 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 6 avril 2021 | Mateo Rosell, A |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 20 20 3529

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-04-2021

| Document brevet cité au rapport de recherche | | Date de publication | Membre(s) de la famille de brevet(s) | | Date de publication |
|---|---|---|---|---|---|
| EP 2371950 | A1 | 05-10-2011 | BR | PI1101054 A2 | 11-12-2012 |
| | | | CA | 2734913 A1 | 26-09-2011 |
| | | | CN | 102234639 A | 09-11-2011 |
| | | | EP | 2371950 A1 | 05-10-2011 |
| | | | ES | 2428825 T3 | 11-11-2013 |
| | | | FR | 2957935 A1 | 30-09-2011 |
| | | | MY | 151988 A | 31-07-2014 |
| | | | PL | 2371950 T3 | 28-02-2014 |
| | | | US | 2011236954 A1 | 29-09-2011 |
| WO 2017174378 | A1 | 12-10-2017 | AU | 2017247922 A1 | 04-10-2018 |
| | | | BR | 112018069674 A2 | 05-02-2019 |
| | | | CA | 3017538 A1 | 12-10-2017 |
| | | | CN | 109072210 A | 21-12-2018 |
| | | | EP | 3440202 A1 | 13-02-2019 |
| | | | FR | 3049957 A1 | 13-10-2017 |
| | | | JP | 2019510500 A | 18-04-2019 |
| | | | US | 2019085310 A1 | 21-03-2019 |
| | | | WO | 2017174378 A1 | 12-10-2017 |
| | | | ZA | 201806072 B | 31-07-2019 |
| WO 2013026964 | A1 | 28-02-2013 | AU | 2012298391 A1 | 30-01-2014 |
| | | | BR | 112014003279 A2 | 01-03-2017 |
| | | | CA | 2840539 A1 | 28-02-2013 |
| | | | CN | 103890171 A | 25-06-2014 |
| | | | DK | 2744899 T3 | 30-05-2016 |
| | | | EP | 2744899 A1 | 25-06-2014 |
| | | | ES | 2571459 T3 | 25-05-2016 |
| | | | FR | 2979111 A1 | 22-02-2013 |
| | | | JP | 6169077 B2 | 26-07-2017 |
| | | | JP | 2014521359 A | 28-08-2014 |
| | | | MX | 349345 B | 25-07-2017 |
| | | | PL | 2744899 T3 | 31-08-2016 |
| | | | UA | 112783 C2 | 25-10-2016 |
| | | | US | 2014302587 A1 | 09-10-2014 |
| | | | WO | 2013026964 A1 | 28-02-2013 |
| | | | ZA | 201400138 B | 30-09-2015 |
| US 2012252066 | A1 | 04-10-2012 | AR | 085754 A1 | 23-10-2013 |
| | | | BR | 112013023986 A2 | 20-09-2016 |
| | | | CA | 2831007 A1 | 04-10-2012 |
| | | | CN | 103492552 A | 01-01-2014 |
| | | | EP | 2691509 A1 | 05-02-2014 |
| | | | JP | 2014513937 A | 19-06-2014 |
| | | | KR | 20140019406 A | 14-02-2014 |
| | | | MX | 356825 B | 15-06-2018 |

EPO FORM P0460

Pour tout renseignement concernant cette annexe : voir Journal Officiel de l'Office européen des brevets, No.12/82

page 1 de 2

## ANNEXE AU RAPPORT DE RECHERCHE EUROPEENNE
## RELATIF A LA DEMANDE DE BREVET EUROPEEN NO.

EP 20 20 3529

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-04-2021

| Document brevet cité au rapport de recherche | Date de publication | Membre(s) de la famille de brevet(s) | | Date de publication |
|---|---|---|---|---|
| | | RU | 2013148010 A | 10-05-2015 |
| | | TW | 201303022 A | 16-01-2013 |
| | | US | 2012252066 A1 | 04-10-2012 |
| | | US | 2018245117 A1 | 30-08-2018 |
| | | WO | 2012135433 A1 | 04-10-2012 |
| DE 102010063942 A1 | 28-06-2012 | AUCUN | | |
| WO 2011019686 A1 | 17-02-2011 | AR | 077838 A1 | 28-09-2011 |
| | | CN | 102574059 A | 11-07-2012 |
| | | DK | 2464446 T3 | 30-03-2015 |
| | | EP | 2464446 A1 | 20-06-2012 |
| | | US | 2012220009 A1 | 30-08-2012 |
| | | WO | 2011019686 A1 | 17-02-2011 |
| WO 2012142557 A1 | 18-10-2012 | AU | 2012242551 A1 | 22-08-2013 |
| | | BR | 112013025614 A2 | 20-09-2016 |
| | | CA | 2832975 A1 | 18-10-2012 |
| | | CN | 103476785 A | 25-12-2013 |
| | | EP | 2697245 A1 | 19-02-2014 |
| | | JP | 2014510796 A | 01-05-2014 |
| | | KR | 20140022839 A | 25-02-2014 |
| | | RU | 2013150782 A | 20-05-2015 |
| | | US | 2015057434 A1 | 26-02-2015 |
| | | WO | 2012142557 A1 | 18-10-2012 |

EPO FORM P0460

Pour tout renseignement concernant cette annexe : voir Journal Officiel de l'Office européen des brevets, No.12/82

page 2 de 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4275167 A
- FR 2555603 B
- EP 2744899 B
- EP 1204738 A

**Littérature non-brevet citée dans la description**

- **HUMBIRD et al.** Process Design and Economics for Biochemical Conversion of Lignocellulosic Biomass to Ethanol. *NREL/TP-5100-57764,* Mai 2011
- **ILMÉN et al.** *Appl.Environ. Microbiol.,* 1997, vol. 63, 1298-1306
- **ALLEN, A.L. ; ANDREOTTI, R.E.** *Biotechnol- Bioengi,* 1982, vol. 12, 451-459
- **MONTENECOURT, B.S. ; EVELEIGH, D.E.** *Appl. Environ. Microbiol.,* 1977, vol. 34, 777-782
- Génétique des microorganismes industriels. **DURAND et al.** Proc. Colloque SFM. 1984, 39-50
- **GABELLE J.-C. ; JOURDIER E. ; LICHT R.B. ; BEN CHAABANE F. ; HENAUT I. ; MORCHAIN J. ; AUGIER F.** Impact of rheology on the mass transfer coefficient during the growth phase of Trichoderma reesei in stirred bioreactors. *Chemical Engineering Science,* 2012, vol. 75, 408-417
- **GABELLE JC ; JOURDIER E ; LICHT RB ; BEN CHAABANE F ; HENAUT I ; MORCHAIN J ; AUGIER F.** Impact of rheology on the mass transfer coefficient during the growth phase of Trichoderma reesei in stirred bioreactors. *Chemical Engineering Science,* 2012, vol. 75, 408-417
- Scale-up agitation criteria for Trichoderma reesei fermentation. **NICOLAS HARDY ; FRÉDÉRIC AUGIER ; ALVIN W. NIENOW ; CATHERINE BÉAI ; FADHEL BEN CHAABANE.** Chemical Engineering Science. Elsevier, 2017, vol. 172, 158-168